**Europäisches Patentamt**

**European Patent Office**

**Office européen des brevets**

(19)

(11) Veröffentlichungsnummer: **0 170 969**
**A1**

(12) **EUROPÄISCHE PATENTANMELDUNG**

(21) Anmeldenummer: **85109171.0**

(22) Anmeldetag: **22.07.85**

(51) Int. Cl.⁴: **A 61 B 17/00**

(30) Priorität: **23.07.84 DE 3427128**

(43) Veröffentlichungstag der Anmeldung: **12.02.86**
Patentblatt 86/7

(84) Benannte Vertragsstaaten: **AT CH IT LI SE**

(71) Anmelder: **Hauer, Gerald Dr., Sintzenichstrasse 2a,**
**D-8000 München 71 (DE)**

(72) Erfinder: **Hauer, Gerald, Dr., Sintzenichstrasse 2a,**
**D-8000 München 71 (DE)**
Erfinder: **Gessler, Reiner, Fabrikstrasse 20,**
**D-8750 Aschaffenburg (DE)**

(74) Vertreter: **Dr. E. Wiegand Dipl.-Ing. W. Niemann Dr. M.**
**Kohler Dipl.-Ing. J. Glaeser Dr. H.-R. Kressin**
**Patentanwälte, Herzog-Wilhelm-Strasse 16,**
**D-8000 München 2 (DE)**

(54) **Venenstripper.**

(57) Ein Venenstripper (1) zur Varizenexstirpation mit einer biegsamen, reißfesten und einen Zuggriff tragenden Sonde (2), an deren freiem Ende ein Exstirpations-Kopf (7) befestigbar ist.

Die Sonde gibt einen durchgehenden Hohlkanal (34), der in oder an der Vorderseite des auf der Führungssonde befestigten Exstirpations-Kopfes (7) mündet.

Durch den Hohlkanal (34) kann beim Einführen der Sonde eine Lösung in die Varize eingespritzt werden, die das Vorschieben der Sonde auch bei einem schwierigen Varizenverlauf erleichtert. Für das Strippen der Vene läßt sich der Exstirpations-Kopf (7) verkantungssicher anbringen.

0170969

Die Erfindung betrifft einen Venenstripper zur Varizenexstirpation mit einer biegsamen, reißfesten und
einen Zuggriff aufweisenden Sonde, an deren freiem
Ende ein Exstirpations-Kopf befestigbar ist.

Zur Beseitigung von Varizen an den unteren Extremitäten
wird häufig das Verfahren der Varizen-Exhairese angewandt. Dazu wird ein Hautschnitt, z.B. in Knöchelhöhe,
zum Aufsuchen der Vena saphena magna bzw. der Vena saphena parva ausgeführt. Die Führungssonde wird dann ohne
den Exstirpations-Kopf in die Vene eingeführt und vorgeschoben, bis zu einem weiter oben liegenden Schnitt, der
z.B. an der Einmündung der Vene in die tiefe Beinvene
liegen kann. Das vordere Ende der Führungssonde wird beim oberen Schnitt herausgezogen. Dann wird gegebenenfalls die Hülle der Führungssonde, die dieser zum Einführen eine ausreichende
Steifigkeit vermittelt hat, von der reißfesten und biegsamen Seele abgezogen und der Exstirpations-Kopf befestigt und in die Vene verlagert. Zuvor kann das obere
Ende der Vene am Exstirpations-Kopf oder an der Seele
befestigt werden. Dann wird die Vene nach unten aus dem
Bein herausgezogen. Das Verfahren läßt sich auch in der
umgekehrten Richtung durchführen.

Aus einem Prospekt "Venenstripper" der Firma B. Braun
Melsungen AG, 3508 Melsungen, herausgegeben im April 1984,
ist ein Venenstripper der eingangs genannten Art bekannt,
bei dem zum Einführen der Sonde eine Führungssonde aufgezogen und festgeschraubt wird, die eine gerundete Spitze
und eine glatte Oberfläche besitzt. Nachdem die Sonde
in die Führungssonde eingeführt und mit ihrem vorderen
Ende aus der Vene herausgeleitet wurde, wird die Führungssonde abgeschraubt und abgezogen und dann der Exstirpations-Kopf aufgeschraubt. Dieser Venenstripper wird auch in dem DE-GM 79 08 610 erläutert. Vorteilhaft ist dabei, daß die dem Zuggriff zugewandte Seite des Exstirpations-Kopfes ausgehöhlt ist, so daß das Umstülpen der herauszuziehenden Vene weitgehend verhindert wird. Nachteilig ist jedoch, daß das ein Gewinde tragende Ende der Sonde vor dem Einführen in die Vene abgedeckt werden muß und daß die Führungssonde sehr klein, unhandlich und auf dem Operationstisch meistens schwer auffindbar ist. Das Gewinde am Ende der Sonde ist aus Kunststoff. Da der Venenstripper verhältnismäßig aufwendig und teuer ist, soll er aus rationellen Gründen mehrmals benutzt werden. Dies ist jedoch kaum möglich, weil beim Sterilisieren der Sonde das aus Kunststoff bestehende Gewinde für die Führungssonde einerseits und den Exstirpations-Kopf andererseits brüchig wird. Der besondere Nachteil des bekannten Venenstrippers liegt darin, daß das Einführen der mit der Führungssonde abgedeckten Sonde schwierig ist, weil permanent die Gefahr besteht, daß sich das vordere Ende der Sonde in Venenwandaussackungen verfängt und die Vene perforiert oder daß sich die Sonde in gewundenen Bereichen der Vene nicht mehr weiterschieben läßt. Es muß dann jeweils von außen eingegriffen werden, was mühsam und für den Patienten unangenehm ist.

Aus der Druckschrift 2-35 der Firma Medimex, 2000 Hamburg 70, ist ein Venenstripper einer anderen Art bekannt, bei dem das Ende der Sonde mit einem kleinen Exstirpations-Kopf einer Minimalgröße, z.B. 9 mm, ausgerüstet ist, der an der Sonde von vornherein festgelegt ist. Zum Venenstripper gehören mehrere vom anderen Ende der Sonde aufsetzbare Scheiben mit unterschiedlich großen Außendurchmessern, z.B. 11 und 15 mm, so daß sich der Venenstripper an die Durchgangsweite der zu entfernenden Vene anpassen läßt. Am dem Exstirpations-Kopf abgewandten Ende der Sonde ist eine spiralförmig vorgeformte Spitze vorgesehen.

Nachteilig ist dabei, daß infolge der ebenen Grundfläche des Exstirpations-Kopfes bzw. der Scheiben permanent die Gefahr besteht, daß die herauszuziehende Vene sich über den Exstirpations-Kopf stülpt. Ein weiterer erheblicher Nachteil ist darin gegeben, daß infolge des von vornherein festgelegten kleinsten Exstiripations-Kopfes auf der Sonde vor dem Einführen der Sonde die richtige Scheibengröße gewählt werden muß. Die spiralförmige Spitze der Sonde ist ungünstig, da die vorgeformten Spiralwindungen kaum jemals dem tatsächlichen Verlauf der Vene entspricht. Im übrigen ist der kleinste Exstirpations-Kopf bereits so groß, daß er für kleine Venen nicht mehr brauch bar ist. Aus den US-PSen 3 659 606 und 3 764 427 ist schließlich ein Venenstripper bekannt, bei dem die Sonde an beiden Enden Zugkörper trägt, was den Vorteil hat, daß der Exstirpations-Kopf sowohl distal als auch proximal befestigt werden kann. Nachteilig ist jedoch, daß das Einführen der Sonde in die Vene schwierig ist, wenn die Vene Wandaussackungen oder starke Krümmungen hat, weil sich die Sonde verfängt und gegebenenfalls die Venenwand perforiert. In diesem Fall ist ein weiterer Schnitt notwendig. Der Exstirpations-Kopf wird dadurch auf der Sonde befestigt, daß er mit einem axial verlaufenden, seitlich offenen Einlegeschlitz quer über die Sonde geschoben und dann zum freien Ende der Sonde bewegt wird, bis der Zugkörper im Exstirpations-Kopf geborgen ist. Dies gestattet zwar die Auswahl des Exstirpations-Kopfes in Anpassung an die Venendurchgangsweite, hat aber gleichzeitig den schwerwiegenden Nachteil, daß der Zugkörper der Sonde beim Herausziehen der Vene leicht aus dem Exstirpation-Kopf herausrutschen kann bzw. daß sich der Exstirpations-Kopf in der Vene verkantet bis die Sonde schließlich seitlich aus dem Einlegeschlitz herausgezwängt wird. Ungünstig ist auch die ebene Grundfläche des Exstirpations-Kopfes, die nicht verhindern kann, daß sich die Vene beim Herausziehen über den Exstirpations-Kopf stülpt, obwohl das Ende der aufgeschnittenen Vene an der Sonde kurz vor dem aufgesetzten Exstirpations-Kopf befestigt wird. Ein verkanteter Exstirpations-Kopf kann nicht nur zu starken Verletzungen im Venenkanal führen ,sondern sich gänzlich von der Sonde lösen, was zum Entfernen des Exstirpations Kopfes einen operativen Eingriff erfordert und die Entfernung der Vene unzulässig verzögert.

Der Erfindung liegt die Aufgabe zugrunde, einen Venenstripper der eingangs genannten Art zu schaffen, mit dem die Varizenexstirpation zügig und störungsfrei ohne die Gefahr stärkerer Verletzungen durchführbar ist. Insbesondere soll angestrebt werden, das Einführen der Sonde in die zu entfernende Vene erheblich zu vereinfachen und dafür Sorge zu tragen, daß beim Entfernen der Vene keine Störungen durch das Umstülpen der Vene über den Exstirpations-Kopf oder durch eine fehlerhafte Verbindung des Exstirpations-Kopfes mit der Sonde auftreten können. Der Venenstripper soll dabei mehrmals wieder verwendbar und beim zwischenzeitlichen Sterilisieren in seiner Gebrauchstüchtigkeit nicht beeinträchtigt werden.

Die gestellte Aufgabe wird gelöst durch einen Venenstripper zur Varizenexstirpation mit einer biegsamen, reißfesten und einen Zuggriff aufweisenden Sonde, an deren freiem Ende ein Exstirpations-Kopf befestigbar ist, der dadurch gekennzeichnet ist, daß die Sonde einen durchgehenden Hohlkanal aufweist, der im oder am freien und zur Befestigung des Exstirpations-Kopfes dienenden Sondenende mündet.

Bei dieser Ausbildung läßt sich beim Einführen und Vorschieben der Sonde Flüssigkeit in die Vene einführen, die entweder eine physiologische Kochsalzlösung oder eine Ringerlösung ist, wodurch sich verhindern läßt, daß sich das vordere Ende der Sonde in Venenwandaussackungen verfängt und die Venenwand perforiert. Auch in stark gewundenen Bereichen der Vene läßt sich durch das Einführen einer Flüssigkeit das Vorschieben der Sonde erleichtern. Die Durchgangsweite des Hohlkanals kann sehr klein sein, weil die für diesen Zweck einzubringende Lösung dünnflüssig ist. Das Einführen der Flüssigkeit erfolgt zweckmäßigerweise mit Druck, so daß die Hindernisse im Verlauf der Vene sanft beiseite gedrückt werden.

Eine zweckmäßige Ausführungsform, bei der eine Kunststoff-Hohlsonde mit einer reißfesten Metall- oder Kunststoff-Seele vorgesehen ist, geht aus Anspruch 2 hervor. Der Hohlkanal ist in der Seele der Sonde angeordnet, die flexibel ausgebildet sein kann und die zum Einführen ausreichende Steifigkeit unter anderem durch die sie umhüllende Kunststoff-Hohlsonde erhält, die zudem mit einer glatten und gleitfreundlichen Oberfläche ausgestattet sein kann.

Eine weitere, zweckmäßige Ausführungsform, bei der die Seele am freien Ende einen im Exstirpations-Kopf formschlüssig festlegbaren Zugkörper trägt, geht aus Anspruch 3 hervor. Der Zugkörper hat nicht nur den Vorteil, daß die verhältnismäßig dünne Sonde keine verletzungsträchtige Spitze hat, sondern sie legt den befestigten Exstirpations-Kopf sicher auf der Sonde fest und gestattet es darüber hinaus, die zum Umfahren von Hindernissen eingeführte Flüssigkeit so günstig zu verteilen, daß die Hindernisse schonend und rasch beiseite gedrückt werden. Eventuell sogar vorgesehene seitliche Auslässe im Zugkörper gestatten es, mit der eingebrachten Flüssigkeit die Vene lokal aufzuweiten oder zu strecken, um den Vorschiebevorgang zu erleichtern.

Eine weitere, zweckmäßige Ausführungsform geht aus Anspruch 4 hervor. In den Anschluß läßt sich eine mit der einzubringenden Flüssigkeit gefüllte Spritze befestigen, mit der - sobald ein fühlbares Hindernis beim Vorschieben der Sonde auftritt - dieses Hindernis leichter umgangen werden kann. Die Spritze kann zweckmäßigerweise während des Gesamteinführvorgangs der Sonde angesteckt bleiben. Der Durchmesser des Hohlkanals beträgt etwa 1 bis 3 mm.

Eine weitere, besonders zweckmäßige Ausführungsform des Erfindungsgegenstandes, bei der der Exstirpations-Kopf einen zentrischen Längskanal für die Sonde aufweist, zu dem ein axialer, seitlich offener Einlegeschlitz führt, wird in Anspruch 5 erläutert. Mit der Sicherung wird der Exstirpations-Kopf so auf der Sonde befestigt, daß er sich beim Herausziehen der Vene nicht verkanten oder sogar von der Sonde abgleiten kann.

Zweckmäßig ist ferner auch das Merkmal von Anspruch 6, weil damit die Befestigung des Exstirpations-Kopfes auf der Sonde durch den Zugkörper, durch welchen beim Einführen der Sonde die Flüssigkeit eingebracht wurde, verbessert wird.

Zweckmäßig ist ferner die Ausführungsform von Anspruch 7. Die konkave Auskehlung des Exstirpations-Kopfes stellt sicher, daß die Vene beim Herausziehen sich nicht über den Exstirpations-Kopf stülpt. Der gerundete Rand vermeidet starke Verletzungen des Venenkanals, selbst wenn dieser stark gewunden verläuft.

Eine weitere, vorteilhafte Ausführungsform geht aus Anspruch 8 hervor. Beim Herausziehen der Vene besteht die größte Gefahr, daß sich die Sonde seitlich aus dem Einlegeschlitz herausbewegt und daß dadurch der Exstirpations-Kopf verkantet wird, nahe dem dem Zuggriff zugewandten Ende des Exstirpations-Kopfes. Da in diesem Bereich die Sicherungsscheibe dank ihres gegenüber dem Einlegeschlitz des Vorderteils verdrehten Einlegeschlitzes das Herausgleiten der Sonde zuverlässig unterbindet, läßt sich die Vene zügig und störungsfrei herausziehen. Die Gefahr, daß sich die Sicherungsscheibe relativ zum Vorderteil verdrehen könnte, ist gering, da die Sicherungsscheibe unter dem Zug beim Herausziehen der Vene gegen den Vorderteil angepreßt wird.

Wichtig ist ferner der Gedanke von Anspruch 9, da durch die Auswahl unterschiedlich großer Sicherungsscheiben eine einfache Anpassung an die jeweilige Venendurchgangsweite auch dann noch möglich ist, wenn die Sonde bereits in die Vene eingeführt ist. Es muß also nicht bereits vor dem Einführen der Sonde eine Auswahl für den zu verwendenden Exstirpations-Kopf getroffen werden, sondern erst zu einem Zeitpunkt, wo die Durchgangsweite der Vene besser bekannt ist.

Zweckmäßig ist auch der Gedanke von Anspruch 10. Die Sicherungsscheibe ist in der verdrehten Stellung am Vorderteil so festgelegt, daß keinesfalls ein Herausgleiten der Sonde aus dem Einlegeschlitz zu befürchten ist. Dabei ist die Befestigung des Exstirpations-Kopfes an der Sonde einfach und mühelos.

Eine weitere, alternative Ausführungsform zeichnet sich gemäß Anspruch 11 aus. Auch das Schnappteil sorgt dafür, daß beim Herausziehen der Vene die Sonde bzw. die Seele nicht seitlich aus dem Einlegeschlitz herausgleiten kann.

Eine weitere, vorteilhafte und alternative Ausführungsform geht aus Anspruch 12 hervor. Der Sperrteil wird nach dem Einlegen der Sonde in den Exstirpations-Kopf umgeklappt und gegebenenfalls verrastet, so daß sichergestellt ist, daß die Sonde beim Herausziehen der Vene nicht seitlich wieder ungewollt aus dem Einlegeschlitz herausgleitet.

Schließlich ist auch noch der Gedanke von Anspruch 13 wichtig, weil der mit dem Herausziehen der Vene eingezogene Drainageschlauch von Verletzungen des Venenkanals herrührendes Blut nach außen ableitet bzw. später das Wundsekret einfach aus dem Venenkanal absaugen läßt. Der Drainageschlauch, der am unteren Ende der Sonde befestigt werden kann, ist so ausgebildet, daß er eine gute Ableitung des Wundsekrets ermöglicht. Dazu ist er z.B. perforiert oder auf seiner Umfangsfläche mit Rillen ausgerüstet, in denen das Wundsekret aus der Venenhöhlung abgeleitet wird. Der Querschnitt des Drainageschlauchs kann auch sternförmig sein.

Der Haltekörper am Ende der Sonde ist vorzugsweise stufenkegelförmig ausgebildet, so daß der Drainageschlauch durch Aufweitung aufgeschoben und somit auf dem Stufenkegel festgelegt werden kann.

Der Haltekörper weist vorzugsweise einen oder mehrere Stege auf, die dazu dienen, ein Verkanten und Verdrehen des auf den Haltekörper aufgeschobenen Exstirpations-Kopfes, der entsprechende Ausnehmungen aufweist, zu verhindern. Der Exstirpationskopf wird über einen seitlichen Schlitz auf die Sonde aufgesetzt und dann auf der Sonde entlang nach unten geschoben, bis der Haltekörper in die Spitze des Exstirpations-Kopfes einrastet, wobei der Exstirpations-Kopf gedreht wird, bis die Stege des Haltekörpers in die entsprechenden Ausnehmungen des Exstirpations-Kopfes einrasten.

Nach einer weiteren Ausgestaltung des Venenstrippers ist vorgesehen, daß ein auswechselbarer Zuggriff auf die Sonde aufschiebbar ist, wobei der Zuggriff eine sich konisch verjüngende Bohrung besitzt, wobei der größte Durchmesser der Bohrung mit dem der Sonde identisch ist und der kleinste Durchmesser der Bohrung wesentlich kleiner ist als der Durchmesser der Sonde, damit der Zuggriff an jeder Stelle der Sonde fixiert werden kann und somit die Belastung jederzeit einen Festsitz auf der Sonde gewährleistet. Auf diese Weise kann insbesondere der Drainageschlauch in einfacher Weise in die Venenhöhlung eingezogen werden.

Anhand der Zeichnungen werden nachstehend Ausführungsformen des Erfindungsgegenstandes erläutert.

Es zeigen:

Figur 1          eine schematische Gesamtansicht eines Venenstrippers,

Figur 2a und 2b   ein vergrößertes Detail des Venenstrippers von Figur 1 in zwei Stellungen,

Figur 3a und 3b   eine weitere Ausführungsform eines Details des Venenstrippers von Figur 1,

Figur 4          ein weiteres Detail in einer Rückansicht,

Figur 5          eine weitere Ausführungsform eines Details,

Figur 6          eine Ausführungsform des Haltekörpers am Ende der Sonde,

Figur 7          einen Querschnitt durch den Haltekörper gemäß Figur 6 entlang der Schnittlinie A-A, und

Figur 8          eine weitere Ausgestaltung eines Haltegriffes.

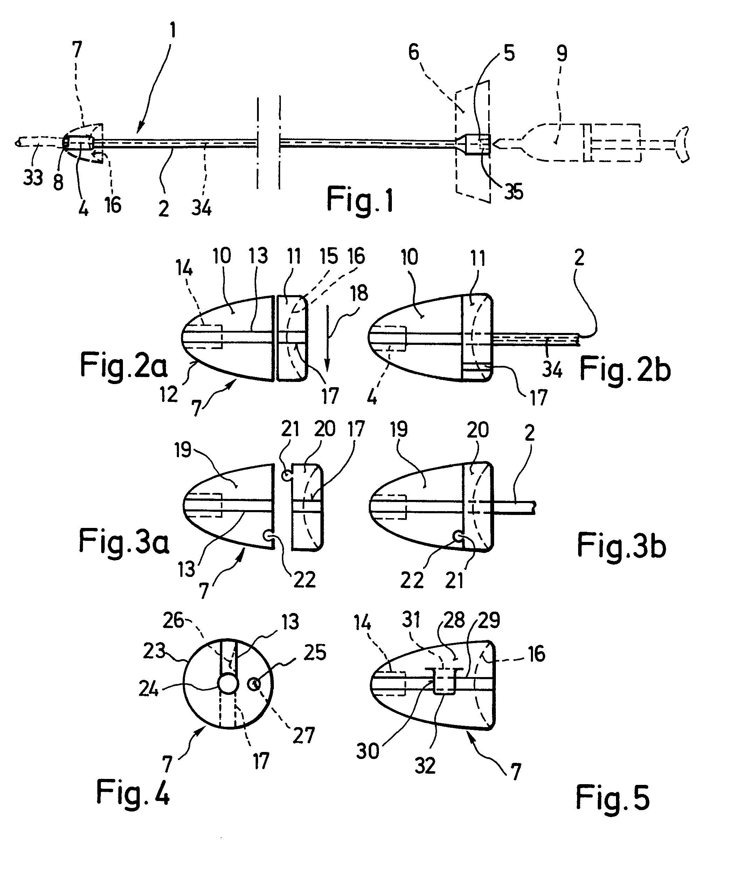

Ein Venenstripper 1 gemäß Figur 1 besteht aus einer Sonde 2 aus einem gewebefreundlichen, glatten Kunststoff, die am vorderen Ende einen Haltekörper 4 und am rückwärtigen Ende einen weiteren Haltekörper 5 trägt. Auf dem rückwärtigen Haltekörper 5 läßt sich ein Zuggriff 6 festlegen, während auf dem Haltekörper 4 und der Sonde 2 am vorderen Ende ein Exstirpations-Kopf 7 festlegbar ist. Im rückwärtigen Haltekörper 5 ist ein Spritzensteckanschluß 35 ausgebildet, in den eine Spritze 9 einsteckbar ist. Die Sonde 2 enthält einen längsdurchgehenden Hohlkanal 34, der vom Spritzensteckanschluß 35 bis zum vorderen, freien Ende des Haltekörpers 4 durchgeht. Im Exstirpations-Kopf 7 bzw. am Haltekörper 4 kann eine Befestigung 8 für einen Drainageschlauch 33 vorgesehen sein. Die dem Zuggriff 6 zugewandte Rückseite des Exstirpations-Kopfes 7 besitzt eine konkave Auskehlung 16. Der Hohlkanal 34 kann durch eine Metall-Seele verstärkt bzw. ausgesteift werden.

In den Figuren 2a und 2b ist eine erste Ausführungsform des in Figur 1 strichliert angedeuteten Exstirpations-Kopfes 7 in einer Seitenansicht dargestellt. Der Exstirpations-Kopf 7 besteht aus einem Vorderteil 10 mit gerundet angespitzter Vorderseite 12 und aus einer zugehörigen Sicherungsscheibe 11, die bei der gezeigten Ausführungsform annähernd den gleichen Außendurchmesser besitzt, wie der Vorderteil 10. Es ist dabei jedoch hervorzuheben, daß dem Vorderteil 10 mehrere Sicherungsscheiben 11 mit unterschiedlichen Außendurchmessern angehören, die in Abhängigkeit von der Durchgangsweite der zu entfernenden Vene wahlweise verwendet werden können.

Der Vorderteil 10 weist einen in Figur 4 angedeuteten, axial durchgehenden Kanal 24 für die Sonde 2 auf, zu dem ein seitlich offener, axialer Einlegeschlitz 13 führt.

Die Sicherungsscheibe 11 besitzt ebenfalls den in Figur 4 angedeuteten Längskanal 24 und einen axial verlaufenden, seitlich offenen Einlegeschlitz 17 zu diesem Kanal. In der Sicherungsscheibe 11 ist ferner die später dem Zuggriff 6 zugewandte Rückseite mit der konkaven Auskehlung 16 versehen, die über eine gerundete Kante 15 in die Außenkontur der Sicherungscheibe 11 übergeht.

Vor der Entfernung der Vene wird die Sonde 2 mit dem Haltekörper 4 in die Vene eingeführt, bis der Haltekörper 4 schließlich am anderen Venenende wieder ins Freie tritt. Beim Vorschieben der Sonde wird mittels einer in den Einsteckanschluß 35 eingesteckten Spritze 9 stets dann eine Flüssigkeit, z.B. eine physiologische Kochsalzlösung oder eine Ringerlösung, durch den Hohlkanal 34 eingeführt, insbesondere dann, wenn ein Schiebewiderstand auftritt. Sobald dann der Haltekörper 4 aus dem anderen Ende der Vene wieder ins Freie tritt, wird der Exstirpations-Kopf 7 befestigt und die Spritze 9 abgezogen. Gleichzeitig wird der Zuggriff 6 aufgebracht.

Das Aufbringen des in den Figuren 2a und 2b gezeigten Exstirpations-Kopfes erfolgt derart, daß zunächst der Vorderteil 10 quer auf die Sonde 2 aufgeschoben wird, bis die Sonde in den Längskanal 24 (Figur 4) eingetreten ist. Danach wird der Haltekörper 4 in die Aufnahme 14 eingeschoben. Im nächsten Schritt wird die Sicherungsscheibe 11 mit ihrem Einlegeschlitz 17 quer über die Sonde 2 geschoben und dann in Richtung eines Pfeiles 18 um zweckmäßigerweise 180° verdreht, so daß die Einlegeschlitze 13 und 17 um 180° zueinander verdreht zu liegen kommen. Dann wird die Sicherungsscheibe 11 an den Vorderteil 10 angepreßt. In Figur 2b ist der Einlegeschlitz 17 der Sicherungsscheibe 11 in einer Zwischenstellung dargestellt.

Die Ausführungsform des Exstirpations-Kopfes 7 gemäß den Figuren 3a und 3b weist wieder einen Vorderteil 19 mit dem Einlegeschlitz 13 sowie eine Sicherungsscheibe 20 mit einem Einlegeschlitz 17 auf. An der dem Vorderteil 19 zugewandten Vorderseite der Sicherungsscheibe 20 ist ein Vorsprung 21 angeformt, der in bezug auf die Sonde 2 und in Drehrichtung der Sicherungsscheibe 20 auf eine Vertiefung 22 des Vorderteils 19 so ausgerichtet ist, daß der Vorsprung 21 in die Vertiefung 22 eingreift, zweckmäßigerweise sogar einrastet, wenn die Sicherungsscheibe 20 so weit relativ zum Vorderteil 19 verdreht worden ist, daß die Einlegeschlitze 13 und 17 nicht mehr aufeinander ausgerichtet sind.

In Figur 4 ist eine weitere Ausführungsform eines Vorderteils 23 des Exstirpations-Kopfes 7 angedeutet, bei der anstelle der Vertiefung 22 der Ausführungsform gemäß Figur 3a in der hinteren Fläche des Vorderteils eine Vertiefung 25 vorgesehen ist, in die ein Zapfen 27 der Sicherungsscheibe eingesteckt werden kann. In Figur 4 ist von der Sicherungsscheibe nur der Einlegeschlitz 17 strichliert angedeutet, der gegenüber dem Einlegeschlitz 13 des Vorderteils 23 um 180° verdreht steht, wenn der Zapfen 27 in die Vertiefung 25 eingesteckt worden ist.

Zusätzlich oder alternativ könnte auch im Einlegeschlitz 13 eine Schnappnase 26 eingeformt sein, über die die Sonde beim Einführen in den Kanal 24 mit Nachdruck hinweggelegt werden muß, und die dafür sorgt, daß die Sonde später nicht mehr aus dem Eilegeschlitz 13 ungewollt herausgleiten kann.

Bei der Ausführungsform gemäß Figur 5 ist ein einstückiger Exstirpations-Kopf 7 vorgesehen, der in seiner Rückseite die konkave Auskehlung 16 besitzt und mit einem längsdurchgehenden Einlegeschlitz 29 versehen ist. Im Körper 28 des Exstirpations-Kopfes 7 ist ein klappenförmiger Sperrteil 30 vorgesehen, der um eine Gelenkstelle 31 quer über oder quer durch den Einlegeschlitz 29 legbar und gegebenenfalls bei 32 verrastbar ist, sobald die Sonde ordnungsgemäß eingeführt worden ist.

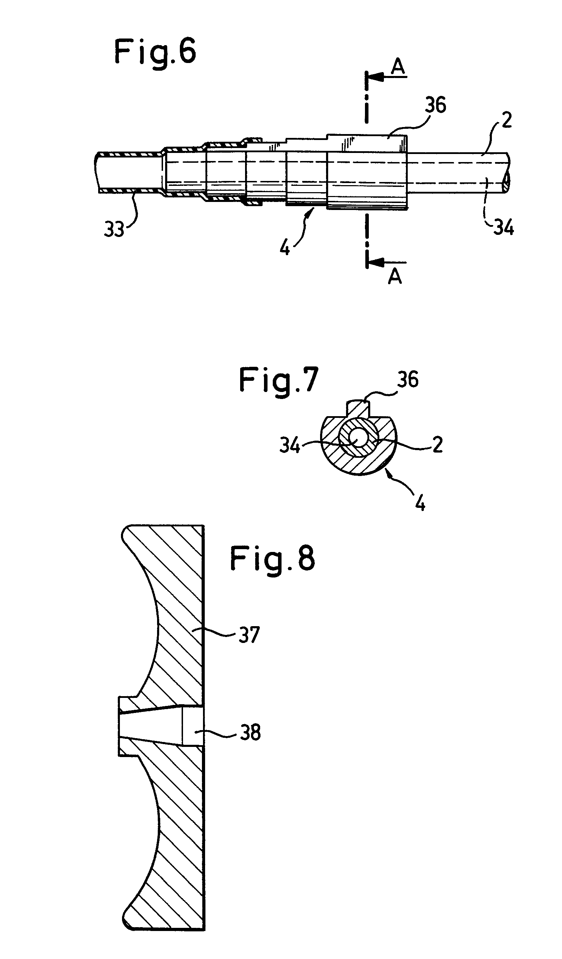

Die Figur 6 zeigt eine Ausführungsform des Haltekörpers 4 am Ende der Sonde 2, wobei der Haltekörper stufenkegelförmig ausgebildet ist. Die stufenkegelförmige Gestalt des Haltekörpers hat den Vorteil, daß ein Drainageschlauch 33 zum Beispiel durch Aufweiten auf den Haltekörper aufgeschoben werden kann und dort auf diese Weise festgelegt wird. Die Sonde 2 ist mit einer Hohlkehle 34 ausgerüstet. Die einzelnen Stufenkegelabschnitte des Haltekörpers sind vorzugsweise so angeordnet, daß die Grundfläche des Kegels der Sonde 2 zugewandt ist und das spitze Ende des Kegels dem aufzuschiebenden Drainageschlauch zugewandt ist. Der Haltekörper weist vorzugsweise 3 bis 6 Einzelstufen auf. Der Haltekörper, z.B. in Form des Stufenkegels, ist weiterhin vorzugsweise so ausgebildet, daß er einen in Axialrichtung ausgerichteten Steg oder mehrere solcher Stege aufweist. Der Steg in dem Haltekörper 4 ist mit 36 bezeichnet (vgl. Figur 7).

Die Figur 7 stellt einen Querschnitt durch den Haltekörper 4 gemäß Figur 6 entlang der Schnittlinie A-A dar. Der Steg 36 dient dazu, ein Verkanten des auf den Haltekörper aufgeschobenen Extirpationskopfes zu verhindern, wobei der Extirpationskopf eine entsprechende Ausnehmung aufweist, in die der Steg 36 einrastbar ist.

Fig. 8 zeigt einen auswechselbaren Haltegriff, der an jeder Stelle der Sonde auf die Sonde aufgeschoben werden kann. Der Haltegriff ist mit 37 bezeichnet. Der Haltegriff 37 weist eine Bohrung 38 auf, die sich konisch verjüngt, wobei der größte Durchmesser der Bohrung mit der der Sonde 2 identisch ist und der kleinste Durchmesser der Bohrung des Zuggriffs wesentlich kleiner ist als der Durchmesser der Sonde 2, damit der Zuggriff an jeder Stelle der Sonde fixiert werden kann und somit bei Belastung jederzeit einen Festsitz auf der Sonde gewährleistet.

Eine weitere Möglichkeit, den Haltegriff auf jeder beliebigen Stelle der Sonde zu befestigen, wird beispielsweise dadurch erreicht, daß der Haltegriff eine Bohrung aufweist, die etwas größer ist als der Außendurchmesser der Sonde (2), und daß an einem Ende der Bohrung mehrere Vorsprünge oder Halteklauen vorgesehen sind, die sich bei Belastung des Haltegriffs auf der Sonde festklemmen. Auf diese Weise wird ein ausreichender Festsitz auf der Sonde gewährleistet.

Patentansprüche

1.    Venenstripper zur Varizenexstirpation, mit einer biegsamen, reißfesten und einen Zuggriff aufweisenden Sonde, an deren freiem Ende ein Exstirpations-Kopf befestigbar ist, d a d u r c h   g e k e n n z e i c h - n e t , daß die Sonde (2) einen durchgehenden Hohlkanal (34) aufweist, der im oder am freien und zur Befestigung des Exstirpations-Kopfes (7) dienenden Sondenende mündet.

2.    Venenstripper nach Anspurch 1, d a d u r c h g e k e n n z e i c h n e t , daß der Hohlkanal (34) durch eine Metall-Seele versteift ist.

3.    Venenstripper nach den Ansprüchen 1 oder 2, d a d u r c h   g e k e n n z e i c h n e t , daß der Hohlkanal (34) einen am vorderen Ende der Sonde (2) vorgesehenen Haltekörper (4) durchsetzt.

4.    Venenstripper nach wenigstens einem der Ansprüche 1 bis 3, d a d u r c h   g e k e n n z e i c h - n e t , daß am hinter dem Zuggriff (6) liegenden Ende der Sonde (2) ein Spritzensteckanschluß (35) zum Hohlkanal (34) vorgesehen ist.

5.    Venenstripper nach wenigstens einem der Ansprüche 1 bis 4, d a d u r c h   g e k e n n z e i c h n e t , daß der Exstirpations-Kopf (7) einen zentrischen Längskanal für die Sonde aufweist, zu dem ein axialer, seitlich offener Einlegeschlitz führt, und eine formschlüssige Sicherung für die eingelegte Sonde (2) aufweist.

0170969

6.　　Venenstripper nach wenigstens einem der Ansprüche 1 bis 5, d a d u r c h   g e k e n n z e i c h n e t , daß der Exstirpations-Kopf (7) eine innenliegende Aufnahme (14) für den Haltekörper (4) aufweist.

7.　　Venenstripper nach wenigstens einem der Ansprüche 1 bis 6, d a d u r c h   g e k e n n z e i c h n e t , daß an der dem Zuggriff (6) zugewandten Seite des Exstirpations-Kopfes (7) eine konkave Auskehlung (16) vorgesehen ist, die mit einem gerundeten Rand (15) in die Außenkontur des Exstirpation-Kopfes (7) übergeht.

8.　　Venenstripper nach wenigstens einem der Ansprüche 1 bis 7, d a d u r c h   g e k e n n z e i c h n e t , daß der Exstirpations-Kopf (7) aus einem Vorderteil (10) und einer Sicherungsscheibe (11) besteht, die mit einem Einlegeschlitz (17) versehen und an der dem Zuggriff (6) zugewandten Seite des Vorderteils (10) auf die Sonde (2) quer aufsteckbar, um die Längsachse verdrehbar und an den Vorderteil (10) anpreßbar ist.

9.　　Venenstripper nach Anspruch 8, d a d u r c h   g e k e n n z e i c h n e t , daß dem Exstirptaions-Kopf (7) mehrere wahlweise benutzbare Sicherungsscheiben (11) mit unterschiedlichen, wirksamen Außendurchmessern angehören.

10.　　Venenstripper nach wenigstens einem der Ansprüche 1 bis 9, d a d u r c h   g e k e n n z e i c h n e t , daß an der Sicherungsscheibe (20) wenigstens ein Vorsprung (21,27) vorgesehen ist, der bei gegenüber dem Vorderteil (19,23) verdrehter Sicherungsscheibe (20) in eine Vertiefung (22,25) des Vorderteils einbringbar, vorzugsweise einrastbar, ist.

11. Venenstripper nach wenigstens einem der Ansprüche 1 bis 9, d a d u r c h   g e k e n n z e i c h n e t , daß im Einlegeschlitz (13) des Exstirpations-Kopfes (7) bzw. des Vorderteils (23) eine Schnappnase (26) vorgesehen ist, über die die Sonde (2) schnappbar ist, und mit der die Sonde gegen ein ungewolltes Herausgleiten aus dem Einlegeschlitz sicherbar ist.

12. Venenstripper nach Anspruch 5, d a d u r c h   g e k e n n z e i c h n e t , daß im Exstirpations-Kopf (7) wenigstens ein nach dem Einlegen der Sonde (2) quer über oder durch den Einlegeschlitz (29) klappbarer, ggf. in umgeklappter Stellung verrastbarer, Sperrteil (30) vorgesehen ist.

13. Venenstripper nach wenigstens einem der Ansprüche 1 bis 12, d a d u r c h   g e k e n n z e i c h n e t , daß am Haltekörper (4) oder am Exstirpations-Kopf (7) eine Befestigung (8) für einen Drainageschlauch (33) vorgesehen ist.

14. Venenstripper nach Anspruch 13, d a d u r c h   g e k e n n z e i c h n e t , daß der Drainageschlauch eine Perforation aufweist oder mit Rillen für die Ableitung des Wundsekrets versehen ist.

15. Venenstripper nach einem der Ansprüche 1 bis 3, d a d u r c h   g e k e n n z e i c h n e t , daß der Haltekörper (4) stufenkegelförmig ausgebildet ist, wobei die Grundfläche des Stufenkegels der Sonde (2) zugewandt ist.

16. Venenstripper nach einem der Ansprüche 1, 3 oder 15, d a d u r c h   g e k e n n z e i c h n e t , daß der

Haltekörper (4) einen oder mehrere Stege aufweist, die ein Verkanten des auf den Haltekörper (4) aufgeschobenen, mit einer dem (oder den) Steg(en) entsprechenden Ausnehmung ausgerüsteten Exstirpations-Kopfes (7) verhindern.

17. Venenstripper nach einem der vorhergehenden Ansprüche, d a d u r c h   g e k e n n z e i c h n e t , daß ein auswechselbarer Zuggriff vorgesehen ist, der eine sich konisch verjüngende Bohrung besitzt, wobei der größte Durchmesser der Bohrung mit dem der Sonde (2) identisch ist, der kleinste Durchmesser der Bohrung wesentlich kleiner ist als der Durchmesser der Sonde (2), damit der Zuggriff an jeder Stelle der Sonde (2) fixiert werden kann, und somit bei Belastung jederzeit einen Festsitz auf der Sonde (2) gewährleistet.

Fig.1

Fig.2a

Fig.2b

Fig.3a

Fig.3b

Fig.4

Fig.5

Fig.6

A

36

2

33

4

34

A

Fig.7

36

34

2

4

Fig.8

37

38

Europäisches Patentamt

**EUROPÄISCHER RECHERCHENBERICHT**

0170969

Nummer der Anmeldung

EP 85 10 9171

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| X | US-A-2 788 787 (TRACE)<br>* Insgesamt * | 1,2,4 | A 61 B 17/00 |
| Y | | 3,5-7,<br>10,13,<br>14 | |
| | --- | | |
| Y | GB-A-1 331 857 (ARMOUR PHARMACEUTICAL CO.)<br>* Abbildungen; Seite 2, Zeilen 1-63 * | 3,5-7,<br>10 | |
| | --- | | |
| D,Y | DE-U-7 908 610 (INTERMEDICAL)<br>* Insgesamt * | 13,14 | |
| A | | 15 | RECHERCHIERTE SACHGEBIETE (Int. Cl.4) |
| | --- | | |
| A | US-A-3 045 676 (SIATEN)<br>* Abbildungen; Anspruch 1 * | 11 | A 61 B |
| | --- | | |
| A | US-A-2 779 334 (SANDBORN)<br>* Abbildungen 3,6; Spalte 2, Zeilen 48-69 * | 12 | |
| | --- | | |
| D,A | US-A-3 764 427 (REIMELS) | | |
| | ----- | | |

Der vorliegende Recherchenbericht wurde für alle Patentansprüche erstellt.

| Rechercheort | Abschlußdatum der Recherche | Prüfer |
|---|---|---|
| DEN HAAG | 01-11-1985 | STEENBAKKER J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82